# EUROPÄISCHE PATENTANMELDUNG

(11) **EP 2 238 898 A1**
(43) Veröffentlichungstag der Anmeldung: **13.10.2010**
(21) Anmeldenummer: 10157881.3
(22) Anmeldetag: 26.03.2010
(51) Int. Cl.: A61B 5/0428, H01P 5/00, A61B 5/055, G01R 33/36, A61N 1/08

(54) **Vorrichtung zur Überwachung eines Lebewesens während eines Magnetresonanz-Experimentes**

(30) Priorität: 30.03.2009 DE 102009001984
(71) Anmelder: Bruker BioSpin AG, 8117 Fällanden (CH)
(72) Erfinder: Schmidig, Daniel, 8200, Schaffhausen (CH)
(74) Vertreter: Kohler Schmid Möbus

(57) **Zusammenfassung**

Eine Vorrichtung zur Überwachung eines Lebewesens während eines Magnetresonanz (= MRI)-Experimentes in einem MRI-Tomographen, wobei die Vorrichtung eine oder mehrere einzelne Elektroden umfasst, die elektrisch leitend mit dem zu untersuchenden Lebewesen verbunden und über Signalleitungen an ein Überwachungsgerät angeschlossen sind, und wobei die Signalleitungen jeweils einzelne Teilstücke umfassen, die über Impedanzen elektrisch miteinander verbunden sind, ist **dadurch gekennzeichnet, dass** die Eigenfrequenzen dieser Teilstücke höher als die NMR-Messfrequenz, vorzugsweise mehr als doppelt so hoch sind, und dass die Teilstücke über frequenzabhängige Impedanzen Zₙ elektrisch miteinander verbunden sind. Damit gelingt es auch einfache Weise, die elektromagnetische Kopplung von der HF-Antenne und den Gradientenspulen auf die Signalleitungen zu minimieren.

## Beschreibung

Die vorliegende Erfindung bezieht sich auf eine Vorrichtung zur Überwachung eines Lebewesens während eines Magnetresonanz (= MRI) -Experimentes in einem MRI-Tomographen, wobei die Vorrichtung eine oder mehrere einzelne Elektroden umfasst, die elektrisch leitend mit dem zu untersuchenden Lebewesen verbunden und über Signalleitungen an ein Überwachungsgerät angeschlossen sind, und wobei die Signalleitungen jeweils einzelne Teilstücke umfassen, die über Impedanzen elektrisch miteinander verbunden sind.

Eine derartige Vorrichtung ist bekannt aus der US 4,951,672 A (=Referenz [7]).

### Vorgeschichte

MRI-Systeme sind weit verbreitete medizinische und diagnostische Geräte. Die primären Komponenten eines MRI-Systems sind der Magnet, welcher ein stabiles und sehr starkes Magnetfeld (B0) erzeugt, die Gradientenspulen, welche ein zusätzliches variables Magnetfeld erzeugen und die HF -Antennen, welche benutzt werden um Energie ins Messobjekt zu senden und um das NMR-Signal aus dem Messobjekt zu empfangen. Ein Computer kontrolliert den gesamten Vorgang und wird benötigt um die empfangene Information zu prozessieren.

Im Folgenden wird das Objekt an welchem die MRI-Messung durchgeführt wird als Patient bezeichnet. Es soll sich dabei jedoch auch ausdrücklich um ein Tier handeln können.

Manchmal ist es nötig den Patienten während einer MRI-Untersuchung zu überwachen. Dies kann einerseits aus medizinischen Gründen nötig sein (z.B. Überwachung der Atmung, Blutsauerstoff, Körpertemperatur) oder weil man die einzelnen Scans des MRI-Experiments mit physiologischen Änderungen im Patienten synchronisieren will (z.B. EKG, EEG oder Atmung).

Im Weiteren werden MRI Experimente, während denen der Patient auf irgendeine Art und Weise überwacht wird als ,überwachte MRI-Experimente' bezeichnet.

Typischerweise werden für überwachte MRI-Experimente Überwachungselektroden am Körper des Patienten befestigt. Die Elektroden sind mit Signalleitungen mit dem Überwachungsgerät verbunden, welches sich häufig außerhalb des elektromagnetisch abgeschirmten MRI-Bereiches befindet und in welchem das von den Elektroden aufgenommene Signal prozessiert und dargestellt wird.

Die Überwachung des Patienten bzw. das Triggern des MRI-Experiments findet während der MRI-Messung statt. D.h. HF-Pulse bzw. Gradientenpulse können auf den Signalleitungen Ströme induzieren. Dies ist insbesondere dann der Fall, wenn die Leitungen direkt durch die HF-Antenne bzw. den Gradienten geführt werden, was häufig unumgänglich ist.

### Kopplung zwischen Gradient und Signalleitungen

In Leiterschleifen (z.B. 1. Elektrode - Körper des Patienten - 2. Elektrode - Äquivalente Impedanz der Signalleitungen) welche von dem sich ändernden Gradientenfeld durchdrungen werden, werden Ströme induziert, welche zu einer Erwärmung (z.B. der Kontaktstelle zwischen Elektrode und Körper) und damit zu Verbrennungen des Patienten führen können.

### Kopplung zwischen HF-Antenne und Signalleitungen

Die Signalleitungen selber wirken direkt als Antennen, welche das elektromagnetische Feld der HF-Antenne empfangen. In diesem Fall spricht man von einer Kopplung zwischen Signalleitungen und HF-Antenne. Je nach Länge und Position der Signalleitungen kann diese Kopplung unterschiedlich stark sein. Im Sendefall, d.h. wenn mit der HF-Antenne gesendet wird, also Leistung ins System abgestrahlt wird, führt eine starke Kopplung zwischen der HF-Antenne und den Signalleitungen dazu, dass in den Signalleitungen große Ströme induziert werden. Dies wiederum führt dazu, dass sich die Signalleitungen erwärmen, was im schlimmsten Fall zu Verbrennungen des Patienten führen kann.

Eine Tatsache, welche in bisherigen Veröffentlichungen zu wenig Beachtung gefunden hat ist die, dass eine Kopplung zwischen der HF-Antenne und Signalleitungen zu B1-Feldverzerrungen in der Nähe der Signalleitungen führt, was Artefakte im MRI-Bild erzeugt oder im Extremfall wiederum zu Verbrennungen des Patienten führen kann. Das ist z.B. dann der Fall, wenn die B1-Feldverzerrung eine lokale Feldüberhöhung im Körper des Patienten erzeugt, was dazu führt, dass an dieser Stelle zu viel HF-Leistung deponiert wird und damit zu viel Wärme erzeugt wird. Im Empfangsfall, d.h. wenn mit der HF-Antenne Signal der angeregten Kernspins empfangen wird. Führt eine Kopplung zwischen HF-Antenne und Signalleitungen wiederum zu einer inhomogenen Ausleuchtung. Im Spezialfall, wo als HF-Antenne eine Phased-array-Spule verwendet wird führt eine Kopplung der einzelnen Array-Spulen zu den Signalleitungen zu einem verstärkten Übersprechen zwischen den einzelnen HF-Kanälen und damit zu einem verschlechterten Signal zu Rausch Verhältnis der MRI-Messung.

### Stand der Technik

**Van Genderingen et al (Radiology 1989)** beschreibt die Verwendung von Kohlefasern als EKG-Leitungen. Das vorherrschende Ziel war es, Feldstörungen des Gradientenfeldes und die damit zusammenhängenden Artefakte, wenn metallische EKG-Kabel verwendet werden, zu reduzieren. Mittlerweile ist die Verwendung von Kohlefaser-EKG-Leitungen weit verbreitet. Der Widerstand von Kohlefaserleitungen liegt im Bereich von einigen hundert Ohm pro Meter. Während dieser Widerstand Bildartefakte aufgrund von Feldstörungen des Gradientenfeldes reduziert, reicht er nicht aus um eine Erwärmung der EKG-Leitungen aufgrund von HF-Strömen zu verhindern oder um die Kopplung zwischen HF-Antenne und EKG-Leitungen ausreichend stark zu unterbinden.

Anlehnend an diese Idee existieren eine Vielzahl von Veröffentlichungen, welche versuchen den Ohmschen Widerstand der Signalleitungen auf die eine oder andere Weise zu erhöhen.

So wird z.B. in den Referenzen [1] - [3] eine Signalleitung beschrieben, welche in einer Dünnschichttechnik hergestellt wird und einen Widerstand von 10'000 Ohm/ft hat. Dabei wird jedoch lediglich auf den verteilten DC-Widerstand der Leitung eingegangen.

Auch Referenz [4] beschreibt eine speziell für Anwendungen im MRI konzipierte EKG-Leitung, welche ein Erwärmen der EKG-Leitung bzw. der Elektrode verhindern soll. Dabei wird als EKG-Leitung ein spiralförmig gewickelter Nickelchrom (Nichrome) - Draht verwendet, womit man einen Widerstand von 7,5 - 30kOhm/Meter erreicht. In diesem Patent wird die Problematik beschrieben, dass, wenn hohe Ohmsche Widerstände in den EKG-Leitungen verwendet werden, diese exakt aufeinander abgestimmt werden müssen, da ansonsten Probleme mit der Gleichtakt-Rauschunterdrückung des EKG-Gerätes entstehen. Die beschriebenen EKG-Kabel werden dadurch sehr aufwändig und teuer.

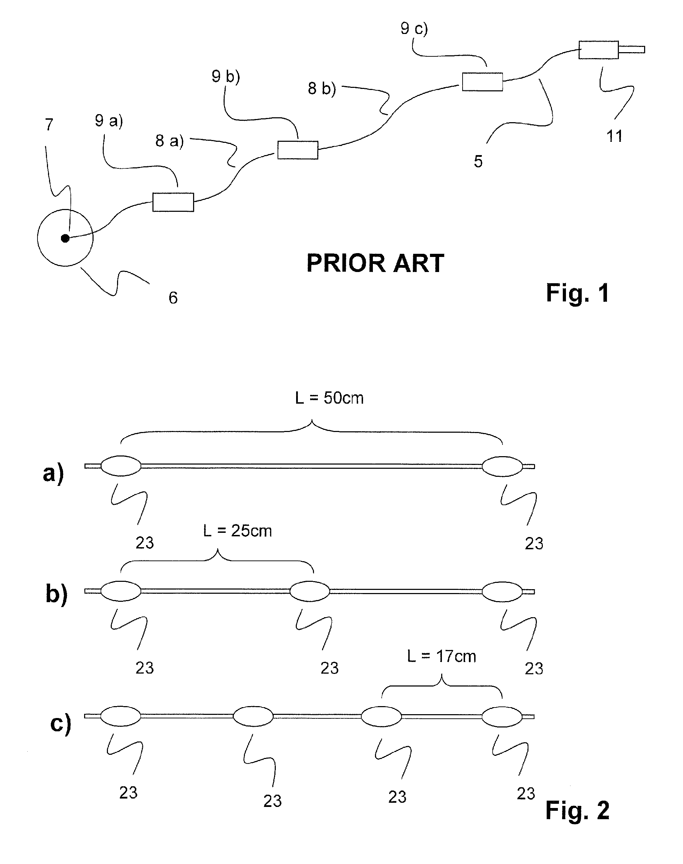

In den Referenzen [5] - [7] wird beschrieben, wie ein oder mehrere diskrete Ohmsche Widerstände in die Signalleitungen eingefügt werden können. So beschreibt Referenz [7] eine speziell für Anwendungen im MRI konzipierte EKG-Leitung, wie in Fig. 1 dargestellt, welche ein Erwärmen der EKG-Leitung bzw. der Elektrode verhindern soll. In Referenz [7] wird behauptet, dass die EKG-Leitung zu diesem Zweck einen hohen DC-Widerstand besitzen muss und beschrieben, wie Widerstände in der Größenordnung von 33-100kOhm in der Elektrode bzw. im EKG-Kabel integriert werden. Das Dokument beschreibt die Möglichkeit den gewünschten Widerstand in kleinere Widerstände aufzuteilen und über die Länge des EKG-Kabels zu verteilen. Die zentrale Bedeutung der Position und der Abstände, in welchen diese Widerstände angebracht werden müssen, wird jedoch nicht erkannt. Zudem ist der Kopplungsmechanismus zwischen EKG-Leitung und HF-Antenne in diesem Patent nicht verstanden.

### Die erfindungsgemäße Vorrichtung

Die erfindungsgemäße Vorrichtung soll der Überwachung eines Lebewesens während eines MRI-Experimentes in einem MRI-Tomographen dienen, wobei die Vorrichtung eine oder mehrere einzelne Elektroden umfasst, die elektrisch leitend mit dem zu untersuchenden Lebewesen verbunden sind und über Signalleitungen an ein Überwachungsgerät angeschlossen sind, und wobei die Signalleitungen einzelne Teilstücke umfassen, die über Impedanzen elektrisch miteinander verbunden sind.

Aufgabe der vorliegenden Erfindung ist es, die elektromagnetische Kopplung von der HF-Antenne und den Gradientenspulen auf die Signalleitungen zu minimieren.

Erreicht wird dieses Ziel auf ebenso überraschend einfache wie wirkungsvolle Weise dadurch, dass die Eigenfrequenzen dieser Teilstücke höher, vorzugsweise mehr als doppelt so hoch wie die NMR-Messfrequenz gewählt werden, und dass die Teilstücke über frequenzabhängige Impedanzen Zₙ elektrisch miteinander verbunden werden.

Eine Minimierung der elektromagnetischen Kopplung zwischen HF-Antenne und Signalleitungen führt zu folgenden Vorteilen:
- Geringere Erwärmung der Signalleitungen und der Elektroden durch die von der HF-Antenne ausgestrahlte HF-Leistung, wodurch das zu untersuchende Lebewesen keine Verbrennungen davonträgt, bzw. weniger stark erwärmt wird.
- Geringere Verschlechterung der magnetischen Feldhomogenität des B1-Sende- bzw. Empfangsfeldes, wodurch weniger zusätzliche Bildartefakte entstehen.
- Geringere HF-Feldüberhöhung im Körper des zu untersuchenden Lebewesens, die von der HF-Antenne stammen und zu Verbrennungen am, bzw. Erwärmung des Lebewesen führen können.
- Geringeres Übersprechen zwischen einzelnen Phased-array-Spulen, das von den Signalleitungen herrührt und zu einer Verschlechterung des Signal-zu-Rausch-Verhältnisses des Empfangssystems führen kann.

Es ist nicht das vordringliche Ziel der vorliegenden Erfindung, Signale, welche durch das MRI-Experiment (HF-Pulse; Gradientenpulse) in den Signalleitungen induziert werden und zu Artefakten im Überwachungssignal führen, zu eliminieren. Dieses Problem kann auf einfache Art und Weise durch Abschirmen und Verdrillen der Signalleitungen gelöst werden. Weiter unten im Text werden jedoch Komponenten beschrieben, welche sowohl HF-Ströme auf den Signalleitungen dämpfen und die Signalleitungen gleichzeitig gegen externe Signale abschirmen.

Durch das einfache Anbringen einer Abschirmung um die Signalleitungen wird das Problem der Kopplung zwischen Signalleitungen und HF-Antenne nicht gelöst, da so die unerwünschten Ströme einfach auf der Abschirmung fließen.

### Definitionen:

- ω:: ω=2πf wobei f die Kernspinresonanzfrequenz ist
- L:: Induktivität einer Spule

Das Ziel, die Kopplung zwischen HF-Antenne und Signalleitungen zu minimieren, wird dadurch erreicht, dass Signalleitungen zwischen Elektroden und Überwachungsgerät in Teilstücke unterbrochen wird, und dass die Teilstücke über Impedanzen miteinander verbunden werden. Dabei wird die Länge der Teilstücke so gewählt ist, dass die Resonanzfrequenz der Teilstücke wesentlich größer als die NMR-Resonanzfrequenz, vorzugsweise mehr als doppelt so hoch, wird und die Impedanz, welche die Teilstücke verbindet, frequenzabhängig ist.

Dadurch, dass die Impedanz frequenzabhängig gewählt wird, existiert die Möglichkeit eine Impedanz zu wählen, welche bei der NMR-Resonanzfrequenz (10 - 1000MHz) sehr hoch ist, bei der Frequenz, welche interessant ist für das Überwachungssignal (<100kHz) jedoch praktisch als Kurzschluss wirkt.

Durch korrekte Wahl der Länge der Teilstücke und der frequenzabhängigen Impedanz kann die Kopplung zwischen den Signalleitungen und der HF-Antennen minimiert werden.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Die Erfindung ist in der Zeichnung dargestellt und wird anhand von Ausführungsbeispielen näher erläutert.

Es zeigen:
- Fig.1: Signalkabel für Überwachungssignal entsprechend Stand der Technik;
- Fig.2a: Kabel bestehend aus Teilstücken der Länge 50cm (λ/2 bei 300MHz);
- Fig.2b: Kabel bestehend aus Teilstücken der Länge 25cm (λ/4 bei 300MHz);
- Fig.2c: Kabel bestehend aus Teilstücken der Länge 16.6cm (λ/6 bei 300MHz);
- Fig.3a: S11 (Reflexionskoeffizient) für Resonanzkreis einer MRI Spule gemäß Experiment 2)a);
- Fig.3b: S11 (Reflexionskoeffizient) für Resonanzkreis einer MRI Spule gemäß Experiment 2)c);
- Fig.3c: S11 (Reflexionskoeffizient) für Resonanzkreis einer MRI Spule gemäß Experiment 2)d);
- Fig.3d: S11 (Reflexionskoeffizient) für Resonanzkreis einer MRI Spule gemäß Experiment 2)e);
- Fig.4: HF-Antenne mit integrierten Signalleitungen;
- Fig.5: Sperrkreis als frequenzabhängige Impedanz, welche das Signalkabel in einzelne Teilstücke unterteilt;
- Fig.6: Induktivität als frequenzabhängige Impedanz, welche das Signalkabel in einzelne Teilstücke unterteilt;
- Fig.7: Gekürzter Bazooka Balun als frequenzabhängige Impedanz, welche das Signalkabel in einzelne Teilstücke unterteilt;
- Fig.8: Bazooka Balun Array als frequenzabhängige Impedanzen, welche das Signalkabel in einzelne Teilstücke unterteilt; und
- Fig.9: Mehrere EKG-Kabel und die Anordnung der einzelnen Teilstücke gegeneinander.

Im Folgenden sei nun zunächst ein einfacher Versuch dokumentiert, welcher die oben gemachten Aussagen erklärt und untermauert.

Dazu wurde der Resonanzkreis einer 300MHz MRI-Spule im unbeladenen Zustand optimal auf 50Ohm abgestimmt. Danach wurden verschiedene freie (ungeerdete) Leitungsstücke in die MRI-Spule eingeführt und das Verstimmen des Resonanzkreises der MRI-Spule dokumentiert (dazu wurde S11 (der Reflexionskoeffizient) des Resonanzkreises gemessen). Anschließend wurde die mit dem Leitungsstück beladene MRI-Spule optimal abgestimmt und die Güte des Resonanzkreises der MRI-Spule bestimmt.

Die Abnahme der Güte zwischen dem unbeladenen und dem beladenen Zustand ist ein direktes Maß für die Kopplung der MRI-Spule zur Leitung in der Spule bzw. zur Leistung welche außerhalb der MRI-Spule d.h. in bzw. über der Leitung dissipiert.

### 1. Einfluss der Länge der einzelnen Teilstücke

Die drei folgenden Kabel werden nacheinander auf exakt dieselbe Position in die MRI-Spule gehalten. Die Teilstücke der Leitungen sind dabei untereinander elektrisch nicht verbunden.
1a) Kabel bestehend aus Teilstücken der Länge 50cm (λ/2 bei 300MHz), Fig. 2a;
1b) Kabel bestehend aus Teilstücken der Länge 25cm (λ/4 bei 300MHz), Fig. 2b;
1c) Kabel bestehend aus Teilstücken der Länge 16.6cm (λ/6 bei 300MHz), Fig. 2c.

**Tabelle 1: Einfluss der Länge der einzelnen Teilstücke**

| | **Q gemessen bei 300MHz** |
|---|---|
| 1a) | Nicht messbar, da Spule nicht abstimmbar |
| 1b) | 69 |
| 1c) | 122 |

Die Güte Q der unbeladenen Spule betrug dabei 125 (gemessen bei 300MHz). Gut erkennbar ist die Tatsache, dass die Kopplung zwischen MRI-Spule und der Leitung massiv von der Länge der Teilstücke abhängt. D.h. die Scheinwiderstände der Impedanzen |Zₙ| zwischen den Teilstücken können beliebig groß gewählt werden (hier: ∞), wenn die Leitungslänge der Teilstücke zu lang ist, bleibt die Kopplung zwischen MRI-Spule und der Signalleitung groß.

### 2. Einfluss der Impedanz Zₙ zwischen den Teilstücken

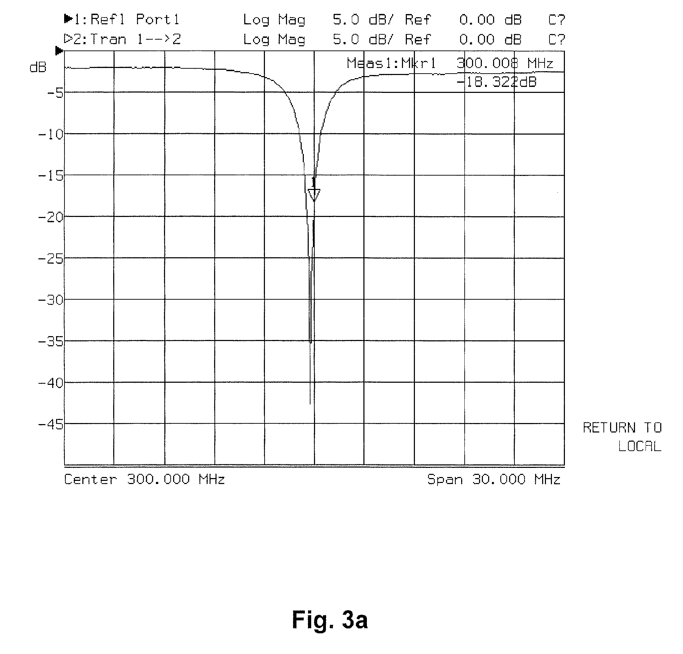

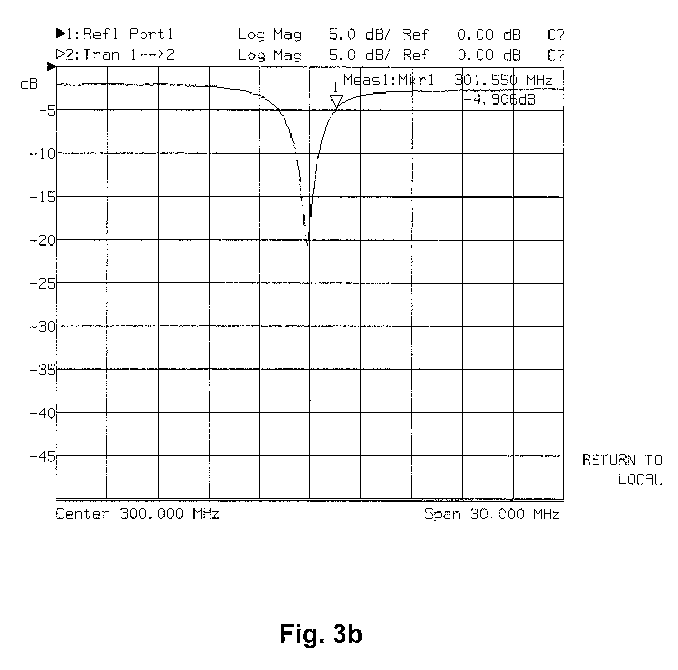

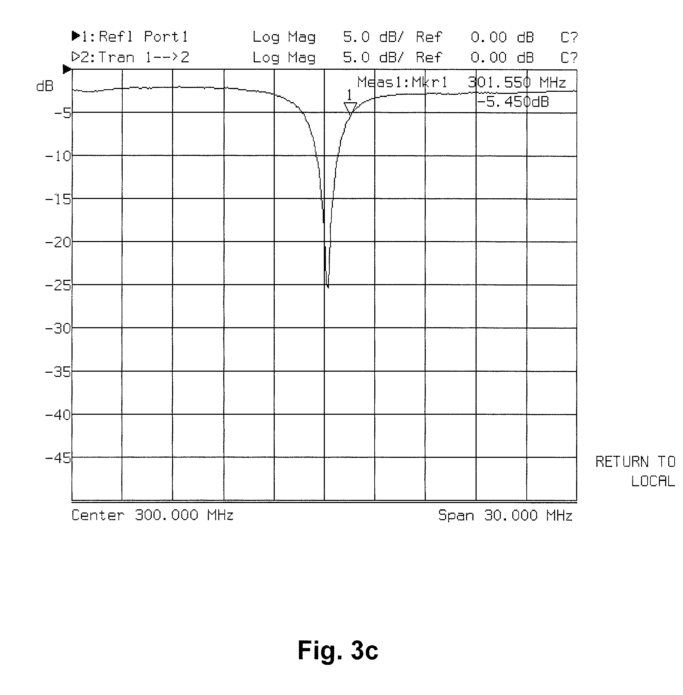

Ein Kabel bestehend aus Teilstücken der Länge 16.6cm (λ/6), entsprechend Fig.2c, wird in die MRI-Spule gehalten. Die Teilstücke sind dabei durch folgende Impedanzen Zₙ verbunden:
2a) Elektrischer Unterbruch (Reflektionskurve dargestellt in Fig.3a)
2b) Ohmscher Widerstand 47Ohm
2c) Induktivität 1.5uH (Reflektionskurve dargestellt in Fig.3b)
2d) Sperrkreis (L=28nH, Q=100) (Reflektionskurve dargestellt in Fig.3c)

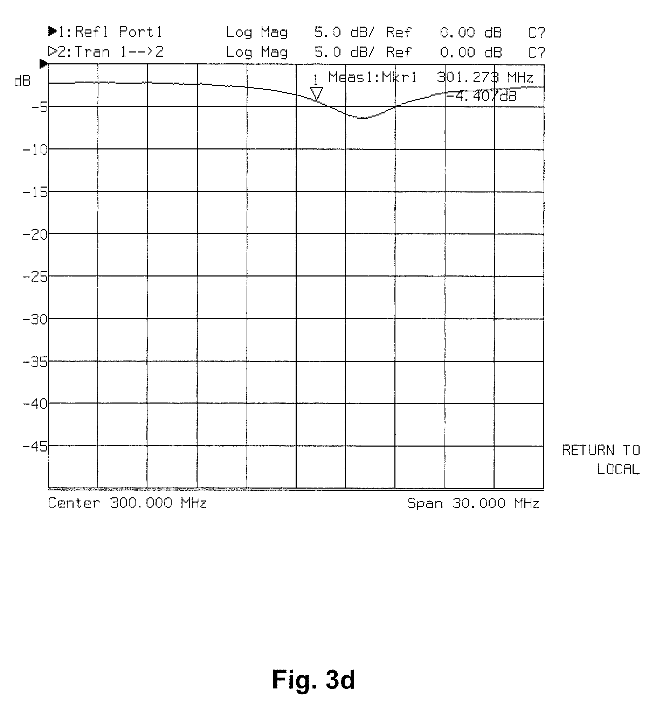

Als Referenz ist noch der Einfluss einer herkömmlichen Kohlefaserleitung (DC-Widerstand: 180Ohm/m) angegeben:
2e) Kohlefaserleitung (Reflektionskurve dargestellt in Fig.3d)

**Tabelle 2: Einfluss der Impedanz zwischen den Teilstücken**

| | **\|Zₙ\| bei 300MHz** | **R** | **Q gemessen bei 300MHz** |
|---|---|---|---|
| 2a) | ∞ | ∞ | 122 |
| 2b) | 47 Ohm | 47 Ohm | Nicht messbar, da Spule nicht abstimmbar |
| 2c) | 2800 Ohm | 0.5 Ohm | 97 |
| 2d) | 5000 Ohm | 0.3 Ohm | 114 |
| 2e) | 1800hm/m | 1800hm/m | 20 |

Gut erkennbar ist die Tatsache, dass die Kopplung zwischen MRI-Spule und Signalleitung stark vom Betrag der Impedanz |Zₙ| des Unterbruchs bei Resonanzfrequenz der MRI-Spule abhängt. Der DC-Widerstand R spielt für die Kopplung keine wesentliche Rolle.

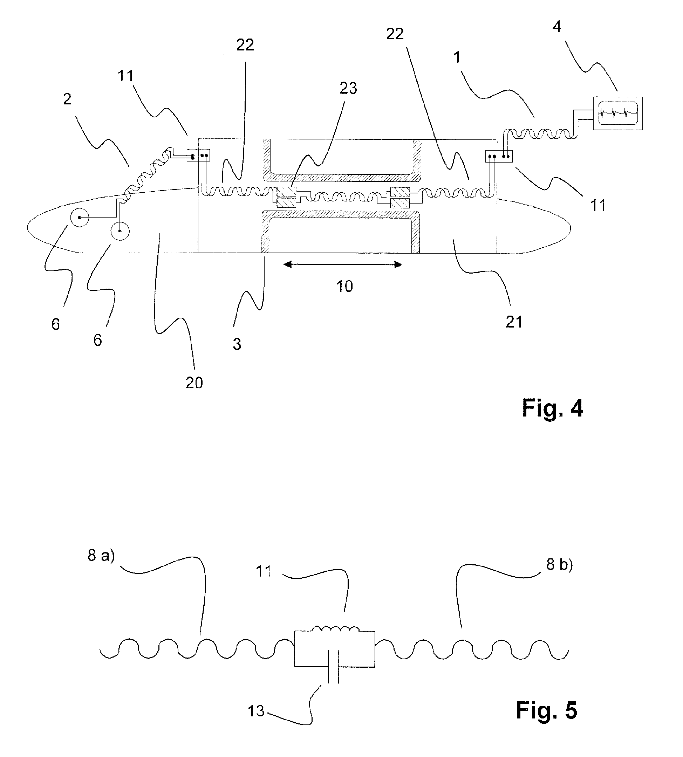

Fig. 4 stellt nun eine erfindungsgemäße Vorrichtung zur Überwachung eines Patienten während der Durchführung von Magnetresonanzmessungen dar. Dabei befindet sich der Patient im MRI-Magneten in der HF-Antenne. Dort wo sich die Signalleitungen innerhalb des Feldes der HF-Antenne befinden sind diese an mehreren Positionen durch frequenzabhängige Impedanzen unterbrochen und unterteilen die Signalleitung in Teilstücke der Länge |<λ/2, vorzugsweise |<λ/4.

Unterschiedliche Ausführungen dieser frequenzabhängigen Impedanzen sind in den Figuren 5 bis 8 dargestellt.

Von Interesse für die Unterdrückung von HF-Strömen auf der Signalleitung sind Vorrichtungen nach obiger Beschreibung, wobei die Blindwiderstände (= Reaktanzen) von Zₙ wesentlich größer sind als deren Ohmsche Widerstände, insbesondere mindestens doppelt so groß, vorzugsweise mehr als zehnmal so groß wie deren Ohmsche Widerstände. Damit wird erreicht, dass der Einfluss auf die hochfrequenten Ströme groß ist, die tieffrequenten Überwachungssignale jedoch durch die Impedanzen Zₙ nicht verändert werden.

Speziell von Interesse sind für die Unterdrückung von HF-Strömen auf der Signalleitung Vorrichtungen nach obiger Beschreibung, wobei die Scheinwiderstände |Zₙ| der frequenzabhängigen Impedanzen Zₙ bei der NMR-Resonanzfrequenz groß (|Zₙ| > 500 Ohm) und bei Frequenzen kleiner als 100kHz klein sind (|Zₙ| < 50 Ohm).

Der Ohmsche Widerstand der Signalleitung kann damit unabhängig von den Impedanzen Zₙ kontrolliert werden, indem z.B. noch zusätzliche rein Ohmsche Widerstände in die Leitung eingebracht werden, oder die Leitung selber aus geeigneten Materialien (z.B. Kohlefaser) hergestellt wird.

In einer Ausführung, dargestellt in Fig. 5, werden für mindestens einen Teil der Impedanzen Zₙ so genannte Sperrkreise verwendet, deren Resonanzfrequenzen auf die NMR-Messfrequenz abgestimmt sind.

Exemplarisch ist hier ein Sperrkreis bestehend aus einer Induktivität und einer parallel dazu geschalteten Kapazität dargestellt. Unter der Voraussetzung, dass die Güte der verwendeten Komponenten genügend groß ist, sieht der HF-Strom in diesem Fall eine Impedanz mit Betrag |Zₙ| ≅ QωL. Der Ohmsche Widerstand entspricht dem einfachen Ohmschen Widerstand der Induktivität. Da diese Sperrkreise selber auf der NMR-Resonanzfrequenz resonant sind, besteht die Gefahr, dass die Sperrkreise selber zur MRI-Spule koppeln. Um das zu verhindern, müssen die Sperrkreise entweder abgeschirmt werden oder so ausgerichtet werden, dass ihr Feld orthogonal zum Feld der MRI-Spule steht.

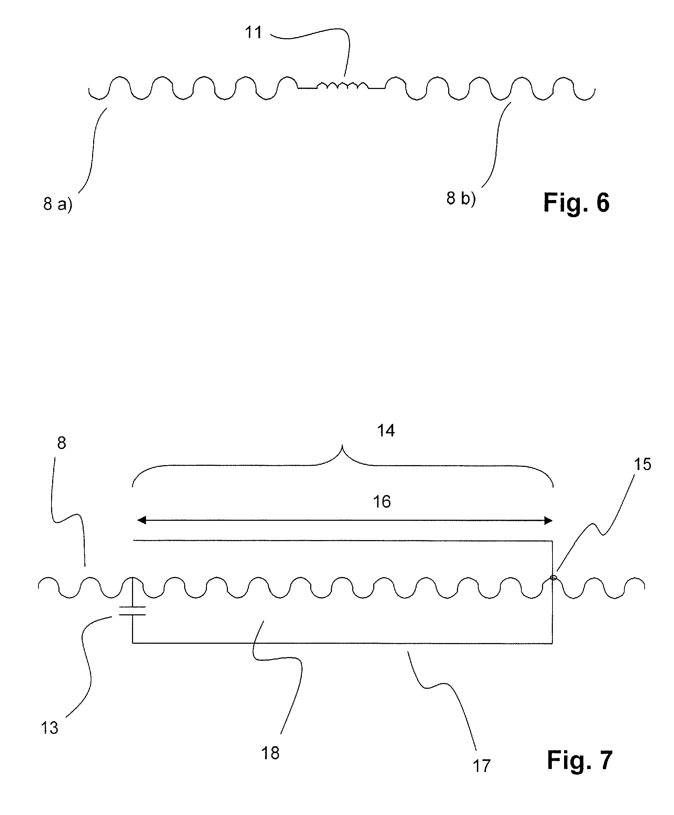

In einer einfacheren Ausführung, dargestellt in Fig. 6, werden für mindestens ein Teil der Impedanzen Zₙ einfache Induktivitäten verwendet. Für den HF-Strom wirkt diese Induktivität als Impedanz mit Betrag |Z| = ωL. Der Ohmsche Widerstand entspricht dem einfachen Ohmschen Widerstand des Drahtes, aus welchem die Induktivität gewickelt ist.

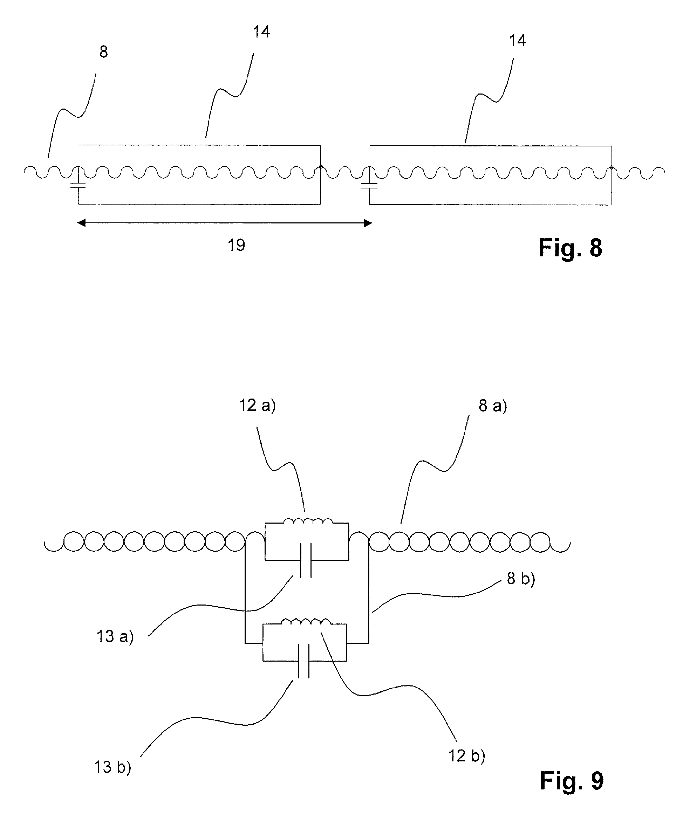

In einer weiteren Ausführung, dargestellt in Fig. 7, werden für mindestens ein Teil der Impedanzen Zₙ «Bazooka Baluns» verwendet, die hinter einander angeordnet sind und in ihrem Innern die Signalleitungen enthalten. Der Vorteil dabei ist, dass die Signalleitung über weite Strecken innerhalb des Außenmantels verläuft und damit zusätzlich gegen die Einkopplung von Signalen in die Signalleitung abgeschirmt ist. Durch Aneinanderreihen mehrere Bazooka Baluns, dargestellt in Fig. 8, kann die Signalleitung so praktisch vollständig abgeschirmt werden.

Alle der beschriebenen HF-Komponenten dämpfen HF-Ströme auf den Signalleitungen und verhindern dadurch eine Kopplung zwischen HF-Antenne und Signalleitungen.

Innerhalb der erfindungsgemäßen Vorrichtung kann es nun von Vorteil sein, die Signalleitungen im Bereich der HF-Antenne des MRI-Empfangssystems auf dem Träger der HF-Antenne mechanisch zu befestigen. Dadurch wird erreicht, dass der verbleibende Einfluss der Signalleitungen auf die HF-Antenne konstant bleibt und innerhalb der HF-Antenne kompensiert werden kann. Zudem kann die Position der Signalleitungen auf dem Träger der HF-Antenne experimentell so gewählt werden, dass die elektromagnetische Kopplung zwischen der HF-Antenne und den Signalleitungen minimal wird.

Aus Fig. 4 ist ersichtlich, dass im Falle, wo die Signalleitungen auf dem Träger der HF-Antenne befestigt sind, Stecker verwendet werden können um die Signalleitungen, welche vom Patienten bzw. vom Überwachungsgerät kommen mit den Signalleitungen, welche am Träger der HF-Antenne befestigt sind elektrisch zu verbinden. Dies hat zur Folge, dass in vielen Fällen, die Präparation des Patienten wesentlich vereinfacht wird, da die Signalleitungen nicht mehr durch die HF-Antenne verlegt werden müssen.

Im Normalfall werden mehr als eine Signalleitung mit dem Patienten verbunden. In diesem Fall ist darauf zu achten, dass Signalleitungen mit gleicher Verteilung der Teilstücke verwendet werden, wobei diese Signalleitungen derart positioniert sind, dass die einander entsprechenden Teilstücke nebeneinander liegen. Andernfalls besteht die Möglichkeit, dass die Impedanz eines Kabels durch das Kabel einer anderen Leitung für HF-Ströme kurzgeschlossen und damit unwirksam wird. Die nebeneinander liegenden Teilstücke der Signalleitungen sollten untereinander verdrillt werden, um Signaleinkopplung in die Signalleitungen zu minimieren. Die beschriebene Anordnung der Impedanzen und Teilstücken der Signalleitung ist für den Spezialfall von zwei Signalleitungen ist in Fig. 9 dargestellt.

### Bezugszeichenliste

- 1: Signalkabel zwischen HF-Antenne und Überwachungsgerät
- 2: Signalkabel zwischen Patient und HF-Antenne
- 3: HF-Antenne
- 4: Überwachungsgerät
- 5: Signalkabel zwischen Überwachungselektrode und Überwachungsgerät
- 6: Überwachungsetektrode
- 7: Kontaktstelle Überwachungselektrode - Signalkabel
- 8: Teilstück des Signalkabels zwischen Überwachungselektrode Überwachungsgerät
- 9: Ohmscher Widerstand, welche die einzelnen Teilstücke voneinander abtrennt
- 10: Distanz zwischen zwei Impedanzen
- 11: Signalkabel Stecker
- 12: Induktivität
- 13: Kapazität
- 14: Bazooka Balun
- 15: Leitende Kontaktstelle zwischen Signalkabel und Außenleiter Bazooka Balun
- 16: Länge des Bazooka Balun
- 17: Außenleiter des Bazooka Balun
- 18: Dielektrikum zwischen Außenleiter und Innenleiter des Bazooka Balun
- 19: Abstand zweier Bazooka Baluns
- 20: Patient
- 21: Träger HF-Antenne
- 22: Auf dem Träger der HF-Antenne befestigtes Signalkabel
- 23: Impedanz, welche die Teilstücke der Signalleitung voneinander abtrennt

### Referenzliste

[1] US 2006/0247509 A1 (ECG cable for use in MRI)
[2] WO 2006/116677 A2
[3] WO 2006/116677 A3 (,search report' zu US 2006/0247509 A1 (ECG cable for use in MRI))
[4] US 6,032,063 A (Distributed resistance leadwire harness assembly for physiological monitoring during magnetic resonance imaging)
[5] JP 4071536 A
[6] US 4,173,221 A (EKG Cable monitoring system)
[7] US 4,951,672 A (Controlled impedance monitoring lead wires)

## Patentansprüche

1. Vorrichtung zur Überwachung eines Lebewesens während eines Magnetresonanz (= MRI) -Experimentes in einem MRI-Tomographen, wobei die Vorrichtung eine oder mehrere einzelne Elektroden umfasst, die elektrisch leitend mit dem zu untersuchenden Lebewesen verbunden und über Signalleitungen an ein Überwachungsgerät angeschlossen sind, und
wobei die Signalleitungen jeweils einzelne Teilstücke umfassen, die über Impedanzen elektrisch miteinander verbunden sind,
**dadurch gekennzeichnet,**
**dass** die Eigenfrequenzen dieser Teilstücke höher als die NMR-Messfrequenz sind, und
**dass** die Teilstücke über frequenzabhängige Impedanzen Zₙ elektrisch miteinander verbunden sind.

2. Vorrichtung nach Anspruch 1, **dadurch gekennzeichnet, dass** die Eigenfrequenzen der Teilstücke mehr als doppelt so hoch wie die NMR-Messfrequenz sind.

3. Vorrichtung nach einem der vorgehenden Ansprüche, **dadurch gekennzeichnet, dass** die Blindwiderstände (= Reaktanzen) der frequenzabhängige Impedanzen Zₙ größer sind als deren Ohmsche Widerstände, insbesondere mindestens doppelt so groß, vorzugsweise mehr als zehnmal so groß wie deren Ohmsche Widerstände.

4. Vorrichtung nach einem der vorgehenden Ansprüche, **dadurch gekennzeichnet, dass** für die Scheinwiderstände |Zₙ| der frequenzabhängigen Impedanzen Zₙ gilt:
- bei der NMR-Resonanzfrequenz |Zₙ| > 500Ohm
- bei Frequenzen klei ner als 100kHz |Zₙ| < 50Ohm.

5. Vorrichtung nach einem der vorgehenden Ansprüche, **dadurch gekennzeichnet, dass** für mindestens einen Teil der Impedanzen Zₙ Sperrkreise verwendet werden, deren Resonanzfrequenzen auf die NMR-Messfrequenz abgestimmt sind.

6. Vorrichtung nach einem der vorgehenden Ansprüche, **dadurch gekennzeichnet, dass** mindestens ein Teil der Impedanzen Zₙ Induktivitäten umfasst.

7. Vorrichtung nach einem der vorgehenden Ansprüche, **dadurch gekennzeichnet, dass** mindestens ein Teil der Impedanzen Zₙ «Bazooka Baluns» umfasst, die hinter einander angeordnet sind und in ihrem Innern die Signalleitungen zu den Elektroden enthalten.

8. Vorrichtung nach einem der vorgehenden Ansprüche, **dadurch gekennzeichnet, dass** die Signalleitungen im Bereich der HF-Antenne des MRI-Empfangssystems am Träger der HF-Antenne mechanisch befestigt sind.

9. Vorrichtung nach Anspruch 8, **dadurch gekennzeichnet, dass** die Position der Signalleitungen auf dem Träger der HF-Antenne experimentell so gewählt wird, dass die elektromagnetische Kopplung zwischen der HF-Antenne und den Signalleitungen minimal wird.

10. Vorrichtung nach einem der vorgehenden Ansprüche, **dadurch gekennzeichnet, dass** mehrere Signalleitungen mit gleicher Verteilung der Teilstücke vorgesehen sind, wobei die Signalleitungen derart positioniert sind, dass die einander entsprechenden Teilstücke nebeneinander liegen.
